Europäisches Patentamt

(19) European Patent Office

Office européen des brevets

(11) Publication number: **0 076 355**
A2

(12) **EUROPEAN PATENT APPLICATION**

(21) Application number: **82101044.4**

(22) Date of filing: **12.02.82**

(51) Int. Cl.³: **A 61 M 1/03**

(30) Priority: **01.10.81 US 307396**

(43) Date of publication of application:
**13.04.83 Bulletin 83/15**

(84) Designated Contracting States:
**AT BE CH DE FR GB IT LI LU NL SE**

(71) Applicant: **ABBOTT LABORATORIES**
**14th Street and Sheridan Road**
**North Chicago, Illinois 60064(US)**

(72) Inventor: **Ramsay, Anne Barlow**
**1111 South Waukegan Road**
**Lake Forest Illinois 60045(US)**

(74) Representative: **Modiano, Guido et al,**
**MODIANO & ASSOCIATI S.A.S. Via Meravigli, 16**
**I-20123 Milan(IT)**

(54) Improved peritoneal dialysis solution.

㊷ An improved peritoneal dialysis solution comprises the conventional electrolyte combination of sodium, calcium, magnesium, chloride, lactate, and sodium hydroxide, the improvement comprising the use of a glucose polymer as the osmotic agent. In a preferred embodiment, the glucose polymer is found in a concentration of from 30 to 125 grams per liter. The large size of the glucose polymer molecules delays their passage through the peritoneum. In addition, the large glucose polymer molecules are slow to be metabolized in the blood. As a result, caloric intake is reduced and the length of time selective osmotic pressure may be applied is increased. Better B.U.N. (Blood Urea and Nitrogen) and creatinine clearances are thereby obtained, and the number of dialyses required can be reduced. Also disclosed is a method of use thereof.

EP 0 076 355 A2

The invention relates generally to the field of medicine and more particularly, to drug-containing solutions for use in performing peritoneal dialysis within a patient.

Several of the functions performed by human kidneys are the removal of waste metabolites, the maintenance of fluid, electrolyte and acid-base balances, and hormone and enzyme synthesis. Loss of normal kidney function (acute or chronic insufficiency) is generally treated through dialysis therapy or transplantation. Transplantation, if successful, restores all the normal functions. Dialysis partially replaces some of the normal kidney functions. However, it does not replace the hormone or enzyme functions, among others, as evidenced by the greatly reduced hemoglobin levels and other changes seen in the blood in chronic uremic patients.

Two types of dialysis therapy are generally employed. In hemodialysis which is most commonly used, the blood is cleansed by passage through an artificial kidney in an extracorporeal membrane system. The waste metabolites diffuse across the membrane and are removed by a washing dialysate solution. Excess fluid is removed by pressure-induced ultrafiltration. The other approach is termed peritoneal dialysis, in which dialysate solution is infused directly into the abdominal cavity. The abdominal cavity is lined by the peritoneal membrane, which is highly vascular. Metabolites are removed by diffusion from the blood across the peritoneal membrane. Excess fluid is removed by osmosis induced by a hypertonic dialysate solution.

In continuous ambulatory peritoneal dialysis a substantially constant presence of dialysate fluid is maintained within a patient at all times, except for that amount of time required to perform an exchange of fluid. Generally, the mean dwell time of the solution is four hours or greater. The steps required in continuous ambulatory peritoneal dialysis comprise: infusing dialysate fluid into the patient's peritoneal cavity; allowing the dialysate fluid to remain in the patient's peritoneal cavity for a specified dwell time during which the patient is ambulatory; exchanging fresh dialysate fluid for spent dialysate fluid after the residence period by draining the spent dialysate fluid from the patient's peritoneal cavity and immediately infusing fresh dialysate fluid into the patient's peritoneal cavity. The exchange of dialysate fluid is repeated several times per day at intervals corresponding to the residence period of time. As a result, a substantially constant presence of dialysis fluid is maintained in the patient and dialysis is occurring essentially continuously for twenty-four hours per day. The contemplated usage of dialysate fluid in the procedure described is approximately 70 liters per week. Normal infusion is approximately 2 to 2-1/2 liters.

Peritoneal dialysis has been conducted in the past using conventional lactate/dextrose or acetate/dextrose solutions of substantially the following components:

|                          | With 1.5% Dextrose | With 2/5% Dextrose | With 4.25% Dextrose |
| --- | --- | --- | --- |
| Dextrose $H_2O$          | 15 g/L             | 25 g/L             | 42.5 g/L            |
| Sodium                   | 132 mEq/L          | 132 mEq/L          | 132 mEq/L           |
| Calcium                  | 3.5 mEq/L          | 3.5 mEq/L          | 3.5 mEq/L           |
| Magnesium                | 1.5 mEq/L          | 1.5 mEq/L          | 1.5 mEq/L           |
| Chloride                 | 102 mEq/L          | 102 mEq/L          | 102 mEq/L           |
| Lactate (or acetate)     | 35 m/eq/L          | 35 m/Eq/L          | 35 mEq/L            |
| Total Osmolarity         | 347 mOsm/L         | 413 mOsm/L         | 486 mOsm/L          |
| Approx. pH               | 5.5                | 5.5                | 5.5                 |

However, the aforementioned dialysate solution, particularly when used in a concentration of 4.25% dextrose (usually used when the patient has become edematous), has been found to provide the patient with an excess quantity of caloric material, resulting in excessive and undesirable weight gain, and other deleterious changes.

Therefore, it is an object of the present invention to provide an improved peritoneal dialysis solution which allows maintenance of selectively increased osmotic pressure without a corresponding increase in caloric intake. It is an additional object of the invention to provide such a solution which is low in cost and easy to manufacture.

1. Protocol

Continuous ambulatory peritoneal dialysis is now an accepted form of dialysis for patients with end stage renal disease. Presently marketed solutions containing glucose as the osmotic agent, however, may not be the ideal. Reports of weight gain and hyperliperdemia among the patient population using these solutions possibly may be attributable to glucose from the dialysate.  Accordingly, alternate osmotic agents such as the present solution are proposed as a replacement for glucose.

2.  Objective

To compare osmotic gradients, serum glucose concentrations, urea and creatinine clearances between a standard glucose containing dialysate and a polymerized glucose containing dialysate in mammals with and without uremia.

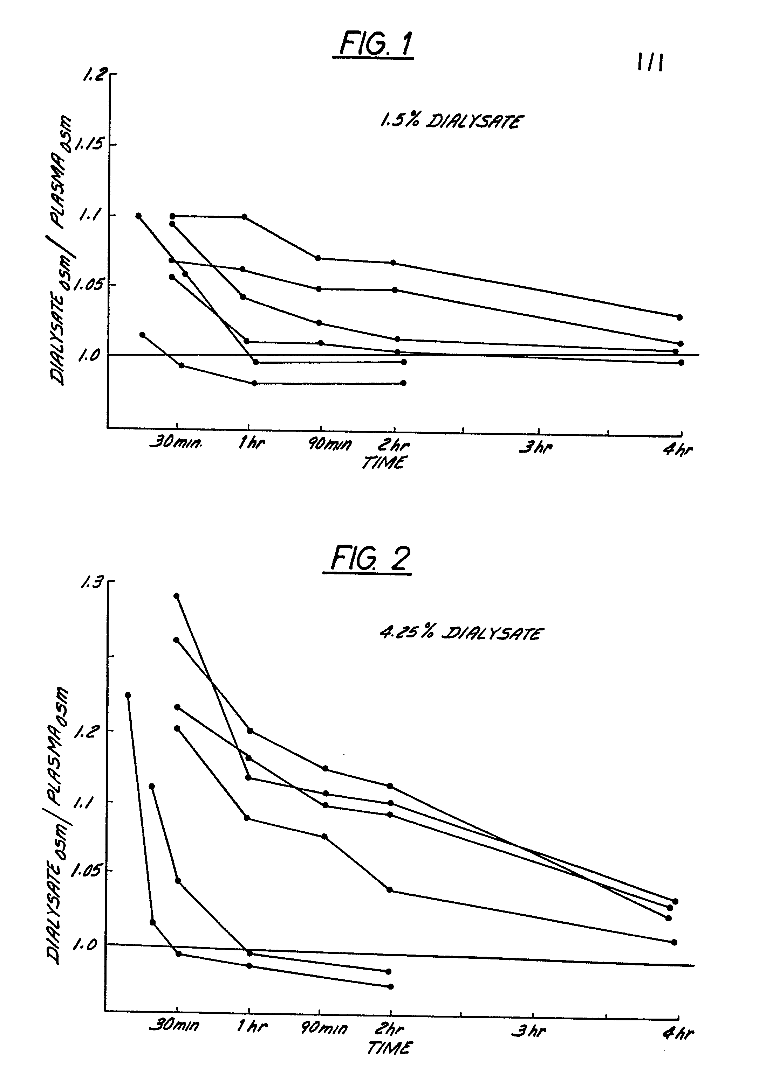

FIGURE 1 of the drawings is a graph illustrating plasma osmolality of peritoneal dialysis patients over a period of four hours after having been administered 2 liters of 1.5% Dextrose Peritoneal Dialysate.

FIGURE 2 of the drawings is a graph illustrating plasma osmolality of peritoneal dialysis patients over a period of four hours after having been administered 2 liters of 4.25% Dextrose Peritoneal Dialysate.

A problem has been found in obtaining adequate clearances in pediatric patients on peritoneal dialysis and in dialyzing all patients on 4.25% dextrose solution.  In order to analyze this problem, a study was performed in the following manner:  Dialysis fluid was put into the abdomen of patients and immediately taken out.  The studies done on this fluid are defined as time 0.  Following that, varying time passes were done as can be seen in the data.  One study which was done was to determine ratio of dialysate to plasma osmolality at varying times.  As can be seen in graph A, at the end of one hour, two of the patients had dialysate fluid which was hypotonic to plasma.  At the end of four hours, in three patients dialysates were hypotonic to plasma (less osmotic pressure than isotonic) and a fourth patient was barely·hypertonic (greater osmotic pressure than isotonic). We determined from this that with 1.5% dextrose dialysate, a number of our young patients could not maintain the necessary osmotic gradient to effectively remove fluid and solute. Graph B shows the same study done with a 4.25% dialysate. Again, two patients were hypertonic at the end of one hour. However, the other four patients did maintain a sufficient osmotic gradient. Table 1 demonstrates the method by which these patients were divided into two groups. The first group, Group A, were those patients who did not maintain a significant osmotic gradient. Group B were those patients who did. Table 2 shows the differences in these groups in regard to age and glucose transfer. The data for glucose transfer is given in milligrams/per minute/per meter squared after a two hour pass with both 1.5% and 4.25% dialysate solution. What is shown is that the patients that did maintain a sufficient osmotic gradient were very young.

The results indicate that osmotic gradient was not maintained because of a much more rapid glucose transfer than in the Group A patients, which were able to dialyze successfully. Table 3 demonstrates the effect of this loss of osmolality on clearance (CL) of urea and the clearance of creatinine. Both of these are measured in milliliters/minutes/meters squared. It can be seen that the patients in Group A had much lower clearances of both these solutes than the patients who maintained good osmotic gradients. Our explanation for this is that the rapid transfer of glucose back into the body causes a solvent drag which results in a return of both urea and creatinine to the body, making dialysis less effective. For these reasons, and also because of the possibility that a high glucose load may cause excessive weight gain to the patient, the present glucose polymer peritoneal dialysis solution has been proposed.

Glucose polymer is not able to enter the circulation as easily as dextrose due to the size of the molecules. In addition, glucose polymer metabolizes more slowly than dextrose. Accordingly, the osmotic gradient of glucose polymers is maintained for a longer period of time. In addition, glucose polymers have been found to be a safe, nontoxic solution when used in I.V. therapy.

## Table 1

**Pt. Group A**

| Dialysate OSM | 1.05 | After 2 Hr. | 4.25% | Pass |
|---------------|------|-------------|-------|------|
| Plasma     OSM | | | | |
| Dialysate OSM | 1.02 | After 2 Hr. | 1.5% | Pass |
| Plasma     OSM | | | | |

**Pt. Group B**

| Dialysate OSM | 1.05 | After 2 Hr. | 4.25 | Pass |
|---------------|------|-------------|------|------|
| Plasma     OSM | | | | |
| Dialysate OSM | 1.02 | After 2 Hr. | 1.5% | Pass |
| Plasma     OSM | | | | |

## Example 1

12.5% Glucose Polymer with Lactate Electrolytes

Glucose Polymer 12.5 g/L

Sodium Lactate 3.92 g/L

Sodium Chloride 5.50 g/L

Calcium Chloride 257.3 mg/L

Magnesium Chloride 152.5 mg/L

Sodium Metabisulfite 300 mg/L

Sodium Hydroxide Q.S. to pH 6.5 before autoclaving

mOsm/kg = 480 equivalent to 4.25% dextrose Titratable acidity, initial after autoclaving 1.0-1.5 ml

## Example 2

6.0% Polymer with Lactate Electrolytes

Glucose Polymer 60 g/L

Remaining electrolytes as above.

Mosm/kg = 340 equivalent to 1.5% dextrose Titratable acidity, initial after autoclaving 1.0-1.5 ml.

Electrolytes for both formulations of Examples 1 and 2:

Sodium 132.0 mEq/L

Calcium 3.5 mEq/L

Magnesium 1.5 mEq/L

Chloride 99.1 mEq/L

Lactate 35.0 mEq/L mEq/L

In this regard, the teachings of U.S. Patent 4,182,756, as signed to the present assignee are hereby incorporated by reference. In the '756 patent are the teachings of several methods of manufacture for a high calorie solution of low molecular weight glucose polymer useful for a safe, nontoxic intravenous administration, and in the present case as a peritoneal dialysate.

In the '756 patent, preferred solutions were characterized as comprising a glucose polymer mixture having an average degree of polymerization between 4 and 6, less than 1% of its molecules greater than 25 glucose units, 4 to 10% of its molecules between 11 and 25 glucose units, 1 to 3% of its molecules 10 glucose units, 3 to 5% of its molecules 9 glucose units, 5 to 8% of its molecules 8 glucose units, 14 to 17% of its molecules 7 glucose units, 16 to 18% of its molecules 6 glucose units, 8 to 9% of its molecules 5 glucose units, 10 to 13% of its molecules 4 glucose units, 13 to 17% of its molecules 3 glucose units, 8 to 13% of its molecules 2 glucose units, and 1 to 3% of its molecules 1 glucose unit. When the dry glucose polymer mixture product obtained from the procedures above were added to distilled water in the amount of 10 to 125 grams per liter, and subjected to steam sterilization, chemical testing of the resulting solution showed that it had an average degree of polymerization of substantially 5, was visually clear when viewed in a light box, and had a glucose polymer distribution of 2.0% glucose, 8.4% maltose, 13.4% maltotriose, 10.5% maltotetraose, 8.7% maltopentaose, 17.3% maltohexaose, 16.4% maltoseptaose, 7.4% maltooctaose, 4.2% maltononaose, 2.8% maltodecaose, 8.8% 11-25 glucose units and 0.7% glucose units greater than 25. The solutions were determined to be stable even during steam sterilization. In the present invention, while the glucose polymer again has an average degree of polymerization of between 4 and 6, the preferred polymeric distribution is as follows:

| Polymer | % Distribution |
|---------|----------------|
| 1 | 0.0 |
| 2 | 0.0 |
| 3 | 24.8 |
| 4 | 14.9 |
| 5 | 19.2 |
| 6 | 30.9 |
| 7 | 4.8 |
| 8 | 2.3 |
| 9 | 1.2 |
| +9 | 1.9 |

In addition to the aforementioned solutions, a wide variety of mono-, di- and tri-saccharide polymers may be used as an osmotic agent in peritoneal dialysis. While dextrose and fructose have been taught in the prior art as being used in peritoneal dialysis, their polymerization and use in peritoneal dialysis is neither taught, suggested, or implied by any known prior art references. Examples of sugars which may be polymerized and used in accordance with the present invention may be seen in the following examples:

Monosaccharides Trioses, D-glyceraldehyde - $(C_3H_6O_3)$ 1,3-dihydroxy-2-propanone;

Tetroses, D-erythrose; $(C_4H_8O_4)$;

Pentoses; L-arabinose - $(C_5H_{10}O_5)$

D-ribose; D-xylose;

Hexoses; D-glucose - $(C_6H_{12}H_5)$;

D-fructose; D-mannose; D-galactose;

Heptoses; sedoheptulose; $(C_7H_{14}O_7)$;

Disaccharides; Sucrose; Lactose; Maltose;

Trisaccharides; Raffinose.

The aforementioned solutions, in order to be suitable for use in human patients must also contain an appropriate blend of electrolytes so as to have a pH of approximately 5.5 and to maintain the stability of the patient during dialysis. In human patients it has been found that the appropriate blend of electrolytes must include sodium of approximately 132 m.Eq./L., calcium of approximately 3.5 m.Eq./L., magnesium of approximately 1.5 m.Eq./L., magnesium of approximately 1.5 m.Eq., chloride of approximately 99 m.Eq./L., lactate or acelate of approximately 35 m.Eq./L., and an osmolality of from 340 to 480 mOsm/L. In a 2-liter quantity of such solution, in one embodiment this would be translated into 392 milligrams of sodium lactate anhydrous, 550 milligrams of sodium chloride, 25.7 milligrams of calcium chloride dihydrate, 15.2 milligrams of magnesium chloride hexahydrate, 30 milligrams of sodium metabisulfite.

The foregoing description merely explains and illustrates the invention and the invention is not limited thereto, except insofar as those skilled in the art who have the disclosure before them are able to make modifications and variations therein without departing from the scope of the invention.

## CLAIMS

1. An autoclavable peritoneal dialysis solution comprising :

10-125 g/liter glucose polymer;

electrolytes comprising approximately: sodium 132-145 m Eq., calcium 3.5-4 m Eq; magnesium 1.5 m Eq., chloride 99-101 m Eq.; lactate 35 m Eq., and sodium hydroxide of from 340 to 485 m mOsm/L for purposes of adjusting the pH of said solution to approximately 5.5.

2. The solution as disclosed in Claim 1 wherein, in each 100 milliliter quantity of said solution, said electrolytes consist essentially of sodium lactate anhydrous 392 mg/100 ml; sodium chloride 550 mg/100 ml; calcium choride dihydrate 25.7 mg/100 ml; magnesium chloride, hexahydrate 15.2 mg/100 ml; and sodium metabisulfite 30 mg/100 ml.

3. The solution as disclosed in Claim 1 wherein said glucose polymer comprises between 1 and 13% glucose polymer, said solution being substantially equivalent to between 1.5 and 4.25% dextrose solution in osmolality, i.e., between approximately 344 and 483.

4. The peritoneal dialysis solution as disclosed in Claim 1 wherein said glucose polymer comprises a glucose polymer mixture having an average degree of polymerization of at least 4 and at least 99% of its molecules less than 26 glucose units, at least 85% of its molecules less than 11 glucose units, and at least 20% of its molecules less than 4 glucose units.

5. A peritoneal dialysis solution containing the following ingredients in the approximate concentration of:

132 m Eq/L sodium

99 m Eq/L chloride

35 m Eq/L lactate

1.5 m Eq/L magnesium

3.5 m Eq/L calcium; and

from 10 to 125 g/L glucose polymers.

6. The solution defined in Claim 1 or 2, or 5 wherein the solution is hypotonic to human blood.

7. The solution defined in Claim 1 or 2 or 5 wherein said glucose polymer mixture has an average degree of polymerization between 4 and 10.

8. The solution defined in Claim 1, 2 or 5 wherein said solution has an average degree of polymerization between 4 and 6, less than 1% of its molecules greater than 25 glucose units, 4 to 10% of its molecules between 11 and 25 glucose units, 1 to 3% of its molecules 10 glucose units, 3 to 5% of its molecules 9 glucose units, 5 to 8% of its molecules 8 glucose units, 14 to 17% of its molecules 7 glucose units, 16 to 18% of its molecules 6 glucose units, 8 to 9% of its molecules 5 glucose units, 10 to 13% of its molecules 4 glucose units, 13 to 17% of its molecules 3 glucose units, 8 to 13% of its molecules 2 glucose units, and 1 to 3% of its molecules 1 glucose unit.

9. An autoclavable peritoneal dialysis solution comprising :

10-125 g/liter glucose polymer;

electrolytes comprising approximately: sodium 132-145 m Eq., calcium 3.5-4 m Eq; magnesium 1.5 m Eq., chloride 99-101 m Eq.; acetate 33 m Eq., and sodium hydroxide of from 340 to 485 m mOsm/L for purposes of adjusting the pH of said solution to approximately 5.5.

10. The solution as disclosed in Claim 1 wherein, in each 100 milliliter quantity of said solution, said electrolytes consist essentially of sodium acetate 271 mg/100 ml; sodium chloride 562 mg/L00 ml; calcium choride dihydrate 25.7 mg/L00 ml; magnesium chloride, hexahydrate 15.2 mg/100 ml; and sodium metabisulfite 27.4 mg/100 ml.

11. The solution as disclosed in Claim 1 wherein said glucose polymer comprises between 1 and 13% glucose polymer, said solution being substantially equivalent to between 1.5 and 4.25% dextrose solution in osmolality, i.e., between approximately 344 and 483.

12. The peritoneal dialysis solution as disclosed in Claim 1 wherein said glucose polymer comprises a glucose polymer mixture having an average degree of polymerization of at least 4 and at least 99% of its molecules less than 26 glucose units, at least 85% of its molecules less than 11 glucose units, and at least 20% of its molecules less than 4 glucose units.

13. A peritoneal dialysis solution containing the following ingredients in the approximate concentration of:

      132 m Eq/1 sodium

       99 m Eq/1 chloride

       35 m Eq/1 lactate

      1.5 m Eq/1 magnesium

      3.5 m Eq/1 calcium; and

      from 10 to 125 g/L glucose polymers.

14. The solution defined in Claims 1 - 13 wherein the solution is hypotonic to human blood.

15. The solution defined in Claims 1 - 14 wherein said glucose polymer mixture has an average degree of polymerization between 4 and 10.

16. The solution defined in Claims 1 - 15 wherein said solution has an average degree of polymerization between 4 and 6, less than 1% of its molecules greater than 25 glucose units, 4 to 10% of its molecules between 11 and 25 glucose units, 1 to 3% of its molecules 10 glucose units, 3 to 5% of its molecules 9 glucose units, 5 to 8% of its molecules 8 glucose units, 14 to 17% of its molecules 7 glucose units, 16 to 18% of its molecules 6 glucose units, 8 to 9% of its molecules 5 glucose units, 10 to 13% of its molecules 4 glucose units, 13 to 17% of its molecules 3 glucose units, 8 to 13% of its molecules 2 glucose units, and 1 to 3% of its molecules 1 glucose unit.

17. The solution as disclosed in Claim 1 or 2 or 3 or 5 or 6 or 16 or 20 or 21 wherein said glucose polymer mixture has the following distribution of polymers:

| Number of Glucose Units | Approximate % Distribution |
|---|---|
| 3 | 24.8 |
| 4 | 14.9 |
| 5 | 19.2 |
| 6 | 30.9 |
| 7 | 4.8 |
| 8 | 2.3 |
| 9 | 1.2 |
| +9 | 1.9. |

0076355

FIG. 1

1.5% DIALYSATE

FIG. 2

4.25% DIALYSATE